# EUROPÄISCHE PATENTSCHRIFT

(11) **EP 0 693 262 B1**
(45) Veröffentlichungstag und Bekanntmachung des Hinweises auf die Patenterteilung: **22.03.2000**
(21) Anmeldenummer: 95111259.8
(22) Anmeldetag: 18.07.1995
(51) Int. Cl.: A45D 24/30, A01K 13/00

(54) **Einrichtung zur Wirkstoff-freien Behandlung parasitenbefallener Hautoberflächen und Haare**
Device for the treatment of parasite infected skin surface and hair, without chemicals
Dispositif pour le traitement de la surface de la peau et des cheveux infectés de parasites sans agents chimiques

(30) Priorität: 22.07.1994 DE 9411937 U
(43) Veröffentlichungstag der Anmeldung: 24.01.1996
(73) Patentinhaber: Trainer, Josef, 6345 Kössen (AT)
(72) Erfinder: Trainer, Josef, 6345 Kössen (AT)

(56) Entgegenhaltungen:
- WO-A-91/15134
- CH-A- 158 666
- FR-A- 1 185 776
- FR-A- 1 249 338
- US-A- 2 474 106
- US-A- 5 261 427

## Beschreibung

Die Erfindung betrifft eine Einrichtung zur Behandlung parasitenbefallener Hautoberflächen von Menschen und Säugetieren.

Eine Einrichtung dieser Art ist aus der US-A-5261427 bekennt und weist folgende Merkmale auf:
ein im wesentlichen rohrförmiges Gehäuse (2), dessen eines Ende durch einen Handgriff (9) verlängert ist und innerhalb dessen eine Heizeinrichtung angeordnet iist, eine Reihe von Kammzinken ist an der Längsseite des Gehäuses und einstückig mit diesem ausgebildet, in einer zu dessen Achse achsparallelen Reihe sowie ihrerseits quer zur Achse und im wesentlichen parallel zueinander angeordnet. Das Gehäuse und die Stutzen sind wärmeisolierend ausgebilde. Im Betrieb wird heiße Luft oder Dampf durch die hohlen Zinken geblasen und soll auf der Kopfhaut Läuse u. dgl. töten; die Zinken bleiben dabei aber verhältnismäßig kühl.

Die FR-A-1249339 offenbart dagegen einen Heizkamm, dessen Gehäuse und Zinken gut wärmeleitfähig sind. Im Gehäuse befindet sich eine Heizeinrichtung. Die Außenoberfläche des Gehäuses ist bei diesem Heizkamm recht warm und im übrigen deutlich wärmer als die Spitzen der Zinken, wie dies zum Ondulieren auch notwendig ist; die Zinken geben dabei den Haaren den erforderlichen Halt, tragen aber zur Wärmebehandlung des Haares nicht bei.

Im Gegensatz zur mechanischen oder chemischen Behandlung ungezieferbefallener, behaarter Körperoberflächen könnte die Bekämpfung des Ungeziefers durch Wärme rasch, aber ohne daß die Gefahr chemischer Schädigung von Haar und Haut vorliegt, doch der obengenannte Heizkamm, der aus seinen Zinken Dampf oder Heißluft ausbläst, kann leicht die Haut flächig verbrennen, wenn sie etwa vom Dampf beaufschlagt wird; ferner umströmt der Dampf die Haare, so daß diese unerwünschterweise verformt werden.

Ausgehend von diesem Stand der Technik liegt der Erfindung die Aufgabe zugrunde, einen Heizkamm zu schaffen, bei dem die obigen Nachteile zumindest abgeschwächt werden.

Erfindungsgemäß wird die Kopfhaut nicht mit einem heißen Gas beaufschlagt, sondern die erhitzten Zinken übernehmen aufgrund ihrer Wärmeabstrahlung die Vernichtung dews Ungeziefers. Dabei basiert die Erfindung ebenso wie der bekannte Heizkamm auf einem anderen Prinzip als die mechanischen Methoden (Abstreifen oder Wegspülen der Parasiten): bei der erfindungsgemäßen Einrichtung werden die Parasiten und auch deren Eier mit den Kammzinken, die durch die Heizeinrichtung erwärmt werden, bis zu einer Temperatur erwärmt, die sie zuverlässig abtötet. Wegen der geringen Größe der Parasiten und erst recht deren Eier einerseits und wegen der geringen Wärmeleitfähigkeit der Haare andererseits genügt es, wenn die erwärmten Kammzinken in die Nähe der Parasiten bzw. der Eier gelangen, um diese abzutöten. Ein körperliches Berühren, Abstreifen oder sonstiges Entfernen der Parasiten ist nicht erforderlich.

Milbenbefallene unbehaarte Hautoberflächen können ebenso mit den Kammzinken überstrichen werden; da diese zwar erwärmt sind, aber nur eine geringe Wärmemenge weiterleiten können, ist eine Verletzung der Haut ausgeschlossen. Diese geringen Wärmemengen reichen aber zum Abtöten der Parasiten vollkommen aus.

Es ist grundsätzlich möglich und gegebenenfalls vorteilhaft, den Kammzinken einen länglich-rechteckigen Querschnitt zu verleihen, wie dies bei den bekannten Läusekämmen der Fall ist, um so den Zinken eine höhere Biegesteifigkeit und Bruchfestigkeit mitzuteilen.

Gemäß einer bevorzugten Ausgestaltung der Erfindung haben aber die Zinken einen im wesentlichen runden Querschnitt, damit sie über ihren Umfang hinweg die im wesentlichen gleiche Temperatur aufweisen und deshalb an jeder Stelle ihres Umfanges imstande sind, Parasiten abzutöten.

Unter einem runden Querschnitt wird auch ein Querschnitt etwa in Form etwa eines regelmäßigen Polygons verstanden; wesentlich ist, daß über den Umfang hinweg keine größeren Temperaturgradienten auftreten.

Es ist auch möglich, einzelne Kammzinken unterschiedlich dick auszubilden, um etwa wärmere und weniger warme Kammzinkengruppen herzustellen, die eine differenziertere Behandlung ermöglichen; gemäß einer weiteren Ausgestaltung der Erfindung weisen aber alle Zinken im wesentlichen den gleichen Querschnitt auf; dies vereinfacht nicht nur die Fertigung der erfindungsgemäßen Einrichtung, sondern gibt dem Benutzer auch die Gewißheit, daß jeder Haut- oder Behaarungsbereich, der einmal während einer gewissen, an jeder Zone der Kammzinkenreihen gleichen Zeitdauer mit irgendwelchen Zinken in Berührung kam, auch in jedem Falle ausreichend behandelt ist.

Ein Nachteil der erwähnten Läusekämme liegt im Umstand, daß die zwischen zwei benachbarten Zinken gebildete Fuge sehr eng ist; sind die Haare an der zu behandelnden Hautoberfläche verfilzt, wie dies etwa nach einem Safaritag ohne weiteres der Fall sein kann, dann ist ein erfolgreiches Durchkämmen der Haare mit einem so feinen Kamm kaum möglich.

Deshalb ist in einer weiteren Ausgestaltung der Erfindung ein recht großer Zinkenabstand gewählt, der letztlich nur so bemessen zu sein braucht, daß die Wärmeabstrahlung zweier benachbarter Kammzinken jeden Parasiten, der sich in einer Kammfuge befindet, auch dann noch zuverlässig abtötet, wenn sich zusätzlich Haare in dieser Kammfuge befinden.

Da die Kammzinken von ihrem einen, in wärmeleitender Verbindung mit der Heizeinrichtung stehenden Ende her erwärmt werden, nimmt ihre Temperatur bis zu ihrem freien Ende hin ab.

Hier wäre es gegebenenfalls zweckmäßig, den der Heizeinrichtung nahen Bereich der Kammzinken mit einem wärmedämmenden Überzug zu versehen, um dafür zu sorgen, daß die freien Enden eine höhere Temperatur erreichen, ohne daß die von der Heizeinrichtung zu erreichende Temperatur erhöht werden muß und ohne daß es somit freiliegende Flächen an den Kammzinken gibt, deren Berührung mit der Haut schmerzhaft oder gar verletzend wäre.

Gemäß einer bevorzugten Weiterbildung der Erfindung wird ein ähnliches Ergebnis jedoch dadurch erreicht, daß die Kammzinken unterschiedlich lang ausgebildet sind; hierbei stellt sich an jedem freien Ende der Kammzinken eine bestimmte Temperatur ein, die umso höher ist, je kürzer die entsprechende Kammzinke ist.

Die längsten Kammzinken dienen gewissermaßen als Abstandshalter, die mit der Oberfläche der behaarten Haut unmittelbar in Eingriff treten und deren Enden die niedrigste Temperatur aufweisen.

Je schräger nun das erfindungsgemäße Gerät gehalten wird, je spitzer demnach der Winkel zwischen den Kammzinken und der Hautoberfläche ist, desto näher kommen die wärmeren Enden der kürzeren Kammzinken der Hautoberfläche. Der Benutzer der erfindungsgemäßen Einrichtung kann somit selbst durch Wahl des Winkels, mit dem der die erfindungsgemäße Einrichtung relativ zur Hautoberfläche hält, deren Erwärmung steuern und somit dafür sorgen, daß nie die Schmerzgrenze erreicht wird.

Es ist aber auch möglich, durch die Wahl unterschiedlich langer Kammzinken deren freie Enden auf einem geometrischen Ort anzuordnen, der keine Gerade ist, sondern eine Krümmung aufweist, die mit der Kontur der Kopfhaut übereinstimmt. Bevorzugt ist in diesem Fall jedoch auch die Heizeinrichtung entsprechend gekrümmt, um zu verhindern, daß die Enden der mittleren Kammzinken eine höhere Temperatur aufweisen als die Enden jener Kammzinken, die an den Enden der Kammzinkenreihe stehen.

Gemäß einer weiteren, bevorzugten Ausgestaltung der Erfindung sind die unterschiedlich langen Kammzinken einzeln oder gruppenweise alternierend angeordnet, so daß etwa auf jede lange Kammzinke eine kurze folgt. Hierdurch wird am besten vermieden, daß versehentlich etwa eine kurze Kammzinke mit ihrem freien, verhältnismäßig heißen Ende versehentlich mit der Haut in Berührung gelangt.

Bevorzugt sitzen die Kammzinken mit ihren der Heizeinrichtung zugeordneten Enden in einer Sockelleiste, die ebenso wie die Kammzinken bevorzugt aus einem Material mit hoher Wärmeleitfähigkeit besteht. Diese Sockelleiste kann dann ihrerseits in unmittelbarer Wärmeleitung mit der Heizeinrichtung stehen. In dieem Fall sorgt die Sockelleiste nicht nur für eine bessere Halterung der Kammzinken, sondern sorgt auch für einen Wärmeausgleich und nivelliert somit Unstetigkeiten in der Wärmeverteilung längs der Heizeinrichtung.

Diese Sockelleiste kann zusammen mit den Kammzinken, die sie trägt, auswechselbar sein und etwa zum Zwecke der Reinigung entnommen werden.

Es ist aber auch möglich, mehrere Sockelleisten mit unterschiedlicher Kammzinkenausbildung bereitzuhalten, die je nach Ausbildung unterschiedliche Wärmecharakteristiken aufweisen und je nach Art und Beschaffenheit der Hautoberfläche, die zu behandeln ist, ausgewählt werden können.

Grundsätzlich ist es möglich, mehrere Kammzinkenreihen vorzusehen, etwa bei der Behandlung langer Haare, die dann wie um einen Frisierstab um die erfindungsgemäße Einrichtung herumgelegt werden und von jeder Kammzinkenreihe unabhängig von den anderen behandelt werden kann. So ist etwa die Behandlung langer Haare schneller und gründlicher möglich als mit einer Einrichtung, die nur eine einzige Kammzinkenreihe vorsieht.

Gemäß einer bevorzugten Ausgestaltung der Erfindung ist jedoch nur eine einzige Kammzinkenreihe vorgesehen, was die Handhabung der erfindungsgemäßen Einrichtung erleichtert und somit höhere Temperaturen an den Kammzinken zuläßt, da die versehntliche Berührung der heißen Kammzinken mühelos vermieden werden kann.

Die Heizeinrichtung kann den Träger selbst bilden; gemäß einer bevorzugten Ausgestaltung ist jedoch der Träger bevorzugt als ein rohrförmiges Gehäuse ausgebildet, das die Heizeinrichtung in ihrem Inneren aufnimmt und das den Erfordernissen entsprechend ausgebildet werden kann.

Es ist grundsätzlich vorteilhaft, das rohrförmige Gehäuse aus einem wärmedämmenden Material herzustellen, das einerseits die Wärmeabgabe auf die Kammzinken beschränkt und somit die Wärmenutzung verbessert, andererseits verhindert, daß die versehentliche Berührung des Gehäuses Verbrennungen verursacht.

Gemäß einer bevorzugen Ausgestaltung der Erfindung ist jedoch ganz im Gegenteil das rohrförmige Gehäuse aus wärmeleitendem Material hergestellt und am einfachsten und besten aus einem Metallrohr gebildet. Hierbei kann nun die nicht mit Kammzinken besetzte Oberfläche dieses Rohres dazu verwendet werden, nicht oder nur kurz behaarte Haut zu behandeln,wobei allerdings die Heizeinrichtung entsprechend abgestimmt sein muß. Außerdem ist es möglich, mit dieser Rohroberfläche über parasitenbefallene Möbeloberflächen, Textilien, Satteldecken usw. zu streichen und somit von vorneherein die Möglichkeit einer Parasitenübertragung zu vermindern oder auszuräumen.

Bevorzugt ist hierbei dann, wenn nur eine einzige Kammzinkenreihe vorgesehen ist, die dieser gegenüberliegende Rohr-Längsseite als Haut-Berührungsabschnitt ausgebildet und zu diesem Zweck bevorzugt ausgebildet, etwa abgeflacht, zur Verbesserung der Wärmeabgabe geschwärzt, mit einer die Temperatur regulierenden und das Gleitverhalten verbessernden Beschichtung versehen usw.. Die Kammzinkenreihe ist dabei vom Haut-Berührungsabschnitt abgewandt, so daß eine versehentliche Berührung der zu behandelten Hautfläche mit jenem Abschnitt der erfindungsgemäßen Einrichtung, der gerade nicht angewandt werden soll, optimal verhindert ist.

Es ist aber gegebenenfalls auch möglich, die Kammzinken zusammen mit ihrer Sockelleiste abzunehmen.

Um bei Anwendung der Kammzinken jede schmerzhafte Berührung mit dem ebenfalls erwärmten Rohr zu vermeinden, ist gemäß einer weiteren, bevorzugten Ausgestaltung der Erfindung eine Hülse aus wärmedämmendem Material vorgesehen, die je nach Bedarf über das Rohr aufgeschoben oder von diesem abgezogen werden kann. Diese Hülse läßt, wenn sie über das Rohr aufgeschoben oder über dieses geklemmt oder geklappt ist, die Zinken frei, deren Temperatur dann bis auf die Gebrauchstemperatur ansteigt, weil die Wärmeabgabe durch die übrige Rohrwand hindurch erschwert ist. Wird die Hülse zum Zweck der Behandlung mittels einer Haut-Berührungsfläche des Rohres abgenommen, dann sinkt die Temperatur der Kammzinken, die dann ohnehin nicht zum Einsatz kommen sollen.

Gemäß einer weiteren, bevorzugten Ausgestaltung weist die Hülse mindestens einen Längsschlitz auf, der ihre Wand durchsetzt und für eine Abstrahlung der Wärme von der Außenoberfläche des Rohres aus sorgt. Diese(r) Längsschlitz(e) sorgt bzw. sorgen für eine gezielte Wärmeabgabe, so daß die den Kammzinken zukommende Wärmemenge durch geeignete Dimensionierung des Schlitzes bzw. der Schlitze eingestellt werden kann.

Besonders wesentlich ist aber der Umstand, daß die Wand der Hülse als Abstandhalter wirkt, so daß dann, wenn das erfindungsgemäße Gerät mit dem Schlitz gegen eine z.B. mit Milben befallene, unbehaarte Hautoberfläche angelegt und über diese hinwegbewegt wird, der unmittelbare Kontakt zwischen Rohr und Haut vermieden ist, aber eine intensive Wärmestrahlung wegen des nur sehr kurzen Abstandes wirksam ist, der im wesentlichen der Wandstärke der Hülse entspricht.

Um diese Wirkung noch zu verstärken, ist der Schlitz als ein an beiden Enden geschlossenes Langloch ausgebildet, um ein Auseinanderspreizen der Schlitzwände zu vermeiden, und erstreckt sich über eine möglichst große Länge.

Die Behandlung der Hautoberfläche mit Wärmestrahlung, gegebenenfalls bei abgenommener Hülse auch mittels Wärmeübertragung, ist nicht nur bei Parasitenbefall sinnvoll, sondern kann z.B. auch bei rheumatischen Schmerzen, Prellungen usw. für Linderung sorgen. Die erfindungsgemäße Einrichtung ist somit als Ausstattung für längere Expeditionen, mehrtägige Jagdexkursionen, Photosafaris usw. auch infolge ihrer universellen Verwendungsmöglichkeiten bestens geeignet.

Um zu vermeiden, daß die Kammzinken bei der Anwendung des Langloches stören, sind sie bevorzugt gegenüber diesem um mindestens 90° versetzt.

Um mit der erfindungsgemäßen Einrichtung eine längere Behandlung durchführen zu können, ist gemäß einer weiteren, bevorzugten Ausgestaltung der Erfindung der Handgriff aus wärmedämmendem Material gebildet oder mit einem solchen Material beschichtet.

Die Heizeinrichtung kann etwa aus einem Glimmstab bestehen, wie er für Handwärmer verwendet wird; je nach Wärmebedarf kann ein solcher Glimmstab an einem oder an beiden Enden entzündet und dann wieder ausgelöscht werden, wenn er nach dem Gebrauch der erfindungsgemäßen Einrichtung nicht aufgebraucht sein sollte. Ein einziger Glimmstab ermöglicht nämlich mehrere Stunden Betrieb.

Mit einer solchen Heizeinrichtung ist somit die erfindungsgemäße Einrichtung von jeglicher Energieversorgung unabhängig betreibbar.

Es ist auch möglich, die Heizeinrichtung mit einem flüssigen Brennstoff wie Katalytbenzin zu betreiben, der in einem Tank untergebracht ist, der im Handgriff angeordnet sein kann.

Eine solche Heizeinrichtung kann auch mit einer Reguliereinrichtung versehen sein, mittels deren die an die Kammzinken und gegebenenfalls die sonstigen Behandlungsoberflächen abzugebende Wärmemenge einstellbar ist. Ein solches Gerät kann etwa in Feldlazaretten, bei Krankenstationen in unterentwickelten Gebieten usw. Anwendung finden, wo eingelieferte Verletzte und Kranke, deren Zustand vorerst keine chemische Entlausung gestattet, vorab mit der erfindungsgemäßen Einrichtung behandelt werden können, um eine Parasitenverseuchung von Krankenbetten usw. möglichst zu verhindern.

Bevorzugt ist jedoch die erfindungsgemäße Heizeinrichtung mit einer elektrischen Heizeinrichtung versehen, die überall dort, wo Batterie- oder Netz-Kraftstrom zur Verfügung steht, wegen der einfachen Anwendbarkeit von Vorteil ist.

Hierbei sind weiter bevorzugt alle Schalt- und Bedienungselemente im Handgriff untergebracht, ausgenommen ein temperaturgesteuerter Sicherheitsschalter, der, soweit er vorgesehen ist, am besten der Heizeinrichtung unmittelbar zugeordnet ist.

Es ist grundsätzlich möglich, im Handgriff der erfindungsgemäßen Einrichtung zur Stromversorgung der Heizeinrichtung eine Batterie- oder Akkuanordnung vorzusehen. Bevorzugt wird die elektrische Heizeinrichtung jedoch über ein Anschlußkabel am besten mittels Netzstromes gespeist.

Dabei ist der Benutzer jedoch gewissermaßen an das Anschlußkabel gefesselt, was zu Ungelegenheiten führen kann.

Um diese auszuräumen, wird gemäß einer weiteren Ausgestaltung der Erfindung vorgeschlagen, der elektrischen Heizeinrichtung einen Wärmespeicher zuzuordnen, der den Betrieb der erfindungsgemäßen Einrichtung über einen gewissen Zeitraum auch ohne Stromversworgung ermöglicht.

Somit kann, wenn der Wärmespeicher aufgeladen ist, das Anschlußkabel von der eigentlichen Stromversorgung gelöst werden, was es dem Benutzer gestattet, sich von dieser mit der heißen Einrichtung zu entfernen, um etwa auf einer naheliegenden Koppel einen parasitenbefallenen Hautabschnitt eines Pferdes zu behandeln.

Diese Ausgestaltung wird dadurch noch verbessert, daß das Anschlußkabel seinerseits an der Einrichtung lösbar anbringbar ist, etwa mittels einer elektrischen Kupplung, die im Handgriff der erfindungsgemäßen Einrichtung angeordnet ist. Somit kann das elektrische Kabel die Handhabung der erfindungsgemäßen Einrichtung nicht beeinträchtigen.

Soweit die erfindungsgemäße Heizeinrichtung mit Strom betrieben iwird, ist es gemäß einer weiteren Ausgestaltung der Erfindung auch vorteilhaft, an der Einrichtung einen Strahler für UVB-Strahlen anzuordnen. Dieser Strahler kann unabhängig von der elektrischen Heizeinrichtung betrieben werden und lenkt seine Strahlung auf den von den Kammzinken behandelten Haar- und Hautbereich.

Die UV-Strahlung unterstützt einerseits die parasitenbekämpfende Wirkung und dient andererseits dazu, den Haarbalg der behandelten Haare zu verbessern.

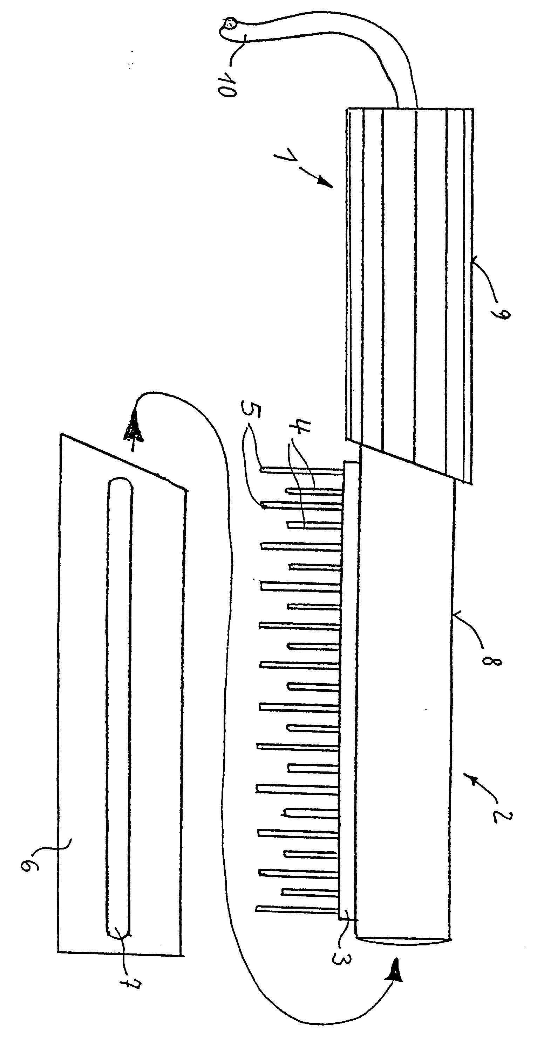

Der Gegenstand der Erfindung wird anhend der beigefügten, schematischen Zeichnung beispielsweise noch näher erläutert, die in ihrer einzigen Figur ein Ausführungsbeispiel einer elektrisch beheizten, erfindungsgemäßen Einrichtung mit abgenommener Hülse zeigt.

Die gezeigte Einrichtung ist aus einem durchgehenden, geraden Metallrohr aufgebaut, dessen erster Abschnitt mit einem Überzug 9 aus wärmedämmendem Material versehen ist und einen Handgriff 1 bildet. Der Überzug 9 ist an seiner Außenoberfläche längsgerippt, um der den Handgriff 1 haltenden Hand einen zuverlässigen Griff zu ermöglichen.

Der Überzug 9 kann von Schalt-, Einstell- und Bedienungselementen durchsetzt sein, die in der Zeichnung der Einfachheit halber weggelassen sind.

Am freien Ende des Handgriffs 1 mündet in diesen ein Stromversorgungskabel 10 ein.

Das dem zweiten, ein Gehäuse 2 bildenden Abschnitt des Rohres zugewandte Ende des Überzugs 9 ist abgeschrägt.

Dieses Gehäuse 2 nimmt in seinem Inneren eine elektrische Heizeinrichtung auf, die in wärmeleitender Verbindung mit einer Metall-Sockelleiste 3 steht, die bevorzugt abnehmbar an der Außenseite des Metallrohres parallel zu dessen Längsachse angebracht ist und sich über nahezu die gesamte Länge des Gehäuses 2 erstreckt.

Die Sockelleiste 3 ist auf der Seite des Gehäuses 2 angeordnet, auf der der kürzeste Teil des abgeschrägten Überzugs 9 des Handgriffs 1 liegt.

Auf der von der Sockelleiste 3 abgewandten Oberfläche des Gehäuses 2 ist dessen Oberfläche als Haut-Berührungsabschnitt 8 ausgebildet.

In der Sockelleiste 3 sitzen alternierend lange Kammzinken 5 und kurze Kammzinken 4 aus geradem Metalldraht, die parallel zueinander und quer zum Gehäuse 2 von der Sockelleiste 3 abstehen.

Alle bisher beschriebenen Metallteile bestehen bevorzugt aus einem Metall mit besonders guter Wärmeleitung, wie etwa Kupfer, Aluminium oder Legierungen dieser Metalle. Das Metall ist bevorzugt mit einer korrosionsbeständigen Beschichtung, etwa Chrom oder Nickel, versehen. Es ist aber auch möglich, die Metallteile oder zumindest die Kammzinken aus rostfeiem Stahl herzustellen.

Eine rohrförmige Hülse 6 aus wärmedämmendem Kunststoff ist so bemessen, daß sie mit leichtem Klemmsitz auf das Gehäuse 2 aufschiebbar ist. Zu diesem Zweck ist die Hülse 6 längs ihrer Erzeugenden mit einer länglichen Aussparung versehen, die sich beim Aufschieben der Hülse 6 auf das Gehäuse 2 um die Sockelleiste 3 herumlegt.

Dabei ist das dem Handgriff 1 zugewandte Ende der Hülse 6 komplementär zum Überzug 9 aus wärmedämmenden Material abgeschrägt.

Hie Hülse weist drei Längsschlitze 7 auf (nur einer ist in der Zeichnung sichtbar), die als Langlöcher mit geschlossenen Enden ausgebildet sind und gegeneinander und gegenüber der länglichen Aussparung jeweils um 90° versetzt sind.

Durch die Längsschlitze hindurch liegt die Oberfläche des Gehäuses 2 frei und kann Wärme abstrahlen.

## Patentansprüche

1. Einrichtung zur Behandlung parasitenbefallener Hautoberflächen von Menschen und Säugetieren, mit den folgenden Merkmalen:
a) ein im wesentlichen rohrförmiges Gehäuse (2), dessen eines Ende durch einen Handgriff (9) verlängert ist und innerhalb dessen eine Heizeinrichtung angeordnet ist,
b) eine Reihe von Kammzinken (4, 5) aus hochwärmeleitfähigem Material, die an der Längsseite des Gehäuses (2) in einer zu dessen Achse achsparallelen Reihe angeordnet sind, sich ihrerseits quer zur Achse und im wesentlichen parallel zueinander erstrecken und in einer Sockelleiste (3) sitzen,
c) die Sockelleiste (3) steht in unmittelbarer Wärmeleitverbindung mit der Heizeinrichtung, und
d) das Gehäuse (2) ist wärmeisolierend ausgebildet oder weist eine abnehmbare Wärmeisolierhülse (6) auf, mit einer länglichen Aussparung, die die Kammzinken (4, 5) oder deren Sockelleiste (3) freiläßt.

2. Einrichtung nach Anspruch 1, dadurch gekennzeichnet, daß die Kammzinken (4, 5) einen im wesentlichen runden Querschnitt aufweisen.

3. Einrichtung nach Anspruch 2, dadurch gekennzeichnet, daß alle Kammzinken (4, 5) im wesentlichen den gleichen Querschnitt aufweisen.

4. Einrichtung nach einem der An sprüche 2 oder 3, dadurch gekennzeichnet, daß der Abstand zwischen benachbarten Kammzinken (4, 5) größer ist als deren Durchmesser.

5. Einrichtung nach einem der Ansprüche 1 bis 4, dadurch gekennzeichnet, daß die Kammzinken unterschiedlich lang sind.

6. Einrichtung nach Anspruch 5, dadurch gekennzeichnet, daß die Kammzinken (4, 5) der Länge nach einzeln oder in Gruppen alternierend angeordnet sind.

7. Einrichtung nach einem der Ansprüche 1 bis 6, dadurch gekennzeichnet, daß nur eine einzige Kammzinkenreihe (4, 5) vorgesehen ist.

8. Einrichtung nach einem der Ansprüche 1 bis 7, dadurch gekennzeichnet, daß mindestens ein sich zum Gehäuse (2) achsparallel erstreckender, der Kammzinkenreihe (4, 5) im wesentlichen gegenüberliegender Abschnitt der Wand des Gehäuses als Haut-Berührungsabschnitt (8) ausgebildet ist, der in wärmeleitender Verbindung mit der Heizeinrichtung steht.

9. Einrichtung nach Anspruch 1, dadurch gekennzeichnet, daß die Wärmeisolierung als eine Hülse (6) aus wärmedämmendem Material, vorzugsweise aus Kunststoff, ausgebildet ist, die auf der Außenseite des Gehäuses (2) anbringbar ist.

10. Einrichtung nach Anspruch 9, dadurch gekennzeichnet, daß die Hülse (6) mindestens einen durchgehenden Schlitz (7) aufweist, der sich parallel zur Achse des Gehäuses (2) erstreckt.

11. Einrichtung nach Anspruch 10, dadurch gekennzeichnet, daß der Schlitz als an seinen Enden geschlossenes Langloch (7) ausgebildet ist, das sich über mehr als drei Viertel der LÄnge des Gehäuses (2) erstreckt.

12. Einrichtung nach einem der Ansprüche 10 oder 11, dadurch gekennzeichnet, daß der Schlitz bzw. das Langloch (7) gegenüber den Kammzinken (4, 5) oder deren Sockelleiste (3) um mindestens 90° versetzt ist.

13. Einrichtung nach einem der Ansprüche 1 bis 12, dadurch gekennzeichnet, daß der Handgriff (1) eine wärmedämmende Beschichtung (9) aufweist oder aus wärmedämmendem Material gebildet ist.

14. Einrichtung nach einem der Ansprüche 1 bis 13, dadurch gekennzeichnet, daß die Heizeinrichtung als elektrische Heizeinrichtung ausgebildet ist.

15. Einrichtung nach Anspruch 14, dadurch gekennzeichnet, daß der elektrischen Heizeinrichtung ein temperaturgesteuerter Sicherheitsschalter zugeordnet ist.

16. Einrichtung nach einem der Ansprüche 14 oder 15, dadurch gekennzeichnet, daß die elektrische Heizeinrichtung ein Wärme-Speicherelement aufweist, das nch seiner Aufheizung einen zeitlich begrenzten Betrieb ohne Anschluß an eine Stromquelle gestattet.

17. Einrichtung nach Anspruch 16, dadurch gekennzeichnet, daß im Handgriff (1) ein elektrische Kupplung angeordnet ist, an der ein Stromversorgungskabel (10) lösbar angebracht ist.

18. Einrichtung nach einem der Ansprüche 14 oder 15, dadurch gekennzeichnet, daß den Kammzinken eine Einrichtung zur Abstrahlung von UVB-Strahlen zugeordnet ist.

## Claims

1. A device for treatment of parasite-infested areas of skin, in humans and mammals, comprising:
a) a carrier having a longitudinal axis and a first end defining a handle and a second end defining a heating means housing,
b) a plurality of comb teath disposed on the external surface of said heating means housing, said comb teeth disposed substantially perpendicular to said longitudinal axis and substantially,
c) the longitudinal axis is adapted to a heat-transfer connection,
d) the housing of a heat-conductive material, said comb teeth disposed substantially perpendicular to said longitudinal axis means for regulating the temperature of said heating means.

2. The device Claim 1, wherein said comb teeth have a substantially round cross-section.

3. The device of Claim 2, wherein the cross-sectional diameters of said comb tetth are substantially equla.

4. The device of Claim 3, wherein the distance between adjacent com teeth is greater than their diameter.

5. The device of Claim 1 to 4, wherein adjacent comb teeth are of different lenght.

6. The Device of Claim 5, further comprising a removable base strip disposed between said heating means housing and said comb teeth.

7. The device of Claim 6, wherein said comb teeth are arranged in a single row.

8. The device of Claims 7, wherein said carrier is substantially tube-shaped. The housing is adapted from a skin contact segment, said contact segment with a heat-transfer connection.

9. The device of claim 8, wherin said heating means housing is comprised of a heat-conductive material.

10. The device of Claim 9, wherin a contact segment disposed substantially opposite.

11. The device of Claim 10, further comprising a removable sleeve having a longitudinal bore and an open first end and a closed second end which can be attached on the outside of said longitudinal bore of said sleeve.

12. The device of Claim 11, wherein the wall of said sleeve is providet with at least one aperture.

13. The device of Claim 12, wherein said aperture is a longitudinal apereture closed at ist ends extending over more than about three-quarters of teh lenght between said first end and said second end of said removable sleeve.

14. The device of Claim 13, wherein said handle is formed of heat-insulating material.

15. The device of Claim 14, wherein said heating means is an electrical heating means.

16. The device to Claim 15, further comprising a temperature-controlled safety switch disposed on sais electrical heating means.

17. The device pursuant to Claim 16, wherein said electrical heating means is providet with a heat storage means to permit operation after said heating means has been disconnected from a power source.

18. The device to Claim 17, wherein the handle is providet with an electrical coupling to which a power supply cable is attached. Further comprising UV radiating means disposed on said second end of said carrier.

## Revendications

1. L'équipment sur le traitement des surfaces à la peau parasitaire de l'hommes et mammifères, avec les marques suivantes:
a) un trognon essentiellement canon (2), qu'il est allongé sur la poignée (9) et en possession de l'installation de chauffage intérieur,
b) une rangée de la roue (4, 5) existant de matériau calorifique, lesquelles se trouve au grand côte du trognon (2) dans une rangée en route parallèle, s'étendre sur l'axe de tangage et l'un à l'autre parallèle essentiellement et quelle sont placé dans le soubassement (3),
c) le soubassement (3) se trouve dans la conduction de chaleur avec l'installation de chauffage, et
d) le trognon (2) est de nature à calorifuge ou il a une calorifugeage amovible (6), avec un évidement oblong, laquelle élargisse la roue (4, 5) ou leur soubassement (3).

2. L'équipment à la revendication 1, caractérise par cela, que les roues (4, 5) présentes une coupe transversale identique.

3. L'équipment à la revendication 2, caractérise par cela, que toutes les roues (4, 5) présentes la coupe transversale identique.

4. L'équipment à une des revendications 2 ou 3, caractérise par cela, que l'écart entre les roues voisines (4, 5) est beaucoup grande que leur diamètre.

5. L'équipment à une des revendications 1 jusque 4, caractérise par cela, que les roues est longes différentes.

6. L'équipment à la revendication 5, caractérise par cela, que les roues (4, 5) sont en possession par rang de longueur ou par groupes alterner.

7. L'équipment à une des revendications 1 jusque 6, caractérise par cela, que une rangée de la roue (4, 5) est unique dans son genre.

8. L'équipment à une des revendications 1 jusque 7, caractérise par cela, qu'il y a une surface de contact (8) de la peau au minimum, lequel s'étendre jusqu'au trognon (2) parallèle au axe et à une rangée de la roue (4, 5) dans le section d'en face de la paroi du trognon, lequel est au assemblage conduction de chaleur avec le chauffage.

9. L'équipment à la revendication 1, caractérise par cela, que le calorifugeage est de nature à la cosse (6) existant de matériau calorifuge, plastique de préférence, laquelle est place à la face extérieure du trognon (2).

10. L'équipment à la revendication 9, caractérise par cela, que la cosse (6) présente une fissure ininterrompu (7), lequel s'étendre jusqu'au axe du trognon (2) parallèle.

11. L'équipment à la revendication 10, caractérise par cela, que la fissure est de nature à la cavité en formation serrée au about (7), laquelle s'étendre jusqu'à plus de trois quarts de la longueur du trognon (2).

12. L'équipment à une des revendications 10 ou 11, caractérise par cela, que la fissure ou bien la cavité en formation (7) en face des roues (4, 5) ou leurs soubassement (3) est transfére contre 90° au minimum.

13. L'équipment à une des revendications 1 jusque 12, caractérise par cela, que la poignée (1) présente un envoi calorifugeage (9) ou il est produit en calorifuge.

14. L'équipment à une des revendications 1 jusque 13, caractérise par cela, que le chauffage est de nature au chauffage électrique.

15. L'équipment à la revendication 14, caractérise par cela, que une serrure de sûreté est coordonnée par le chauffage électrique.

16. L'équipment à une des revendications 14 ou 15, caractérise par cela, que le chauffage électrique présente une capacité de calorique, laquelle permit une marche limité temporelle sans la distribution à la source d'électricité d'après puissance calorifique.

17. L'équipment à la revendication 16, caractérise par cela, que dans la poignée (1) est une couplage électrique, ce qu'elle est fixé détachable un câble pour l'alimentation (10).

18. L'équipment à une des revendications 14 ou 15, caractérise par cela, que les roues est coordonnées un dispositif pour l'émission du UVB-rayonnement.
